# EUROPEAN PATENT APPLICATION

(11) **EP 2 186 907 A1**
(43) Date of publication of application: **19.05.2010**
(21) Application number: 09006623.4
(22) Date of filing: 15.05.2009
(51) Int. Cl.: C12Q 1/68

(54) **X-chromosomal variation as a diagnostic and therapeutic marker for the progression to AIDS**

(30) Priority: 11.11.2008 US 113342 P
(71) Applicant: Deutsches Primatenzentrum GmbH, 37077 Göttingen (DE); Leibniz-Institut für Altersforschung Fritz-Lipmann-Institut e.V. (FLI), 07745 Jena (DE)
(72) Inventor: Siddiqui, Roman, 37075 Göttingen (DE); Sauermann, Ulrike, 37073 Göttingen (DE); Altmüller, Janine, 51399 Burscheid (DE); Nürnberg, Peter, 16818 Neuruppin OT Wuthenow (DE); Fritzer, Elfriede, 24214 Gettorf (DE); Nothnagel, Michael, 24116 Kiel (DE); Krawczak, Michael, 24113 Rammsee (DE); Platzer, Matthias, 07743 Jena (DE)
(74) Representative: Krauss, Jan

(57) **Abstract**

The present invention relates to a method for diagnosing the likelihood and/or speed of AIDS progression in a mammal, comprising the analysis of at least one of the X-chromosomal SNPs from the *DXS986* region, and preferably rs5968255. Another aspect of the present invention relates to a method for treating AIDS progression in a mammal based on said diagnosis. Other aspects include a method for screening for an anti-AIDS progression pharmaceutical compound, a pharmaceutical composition for the treatment of AIDS and/or AIDS progression, and a diagnostic kit.

## Description

The present invention relates to a method for diagnosing the likelihood and/or speed of AIDS progression in a mammal, comprising the analysis of at least one of the X-chromosomal SNPs from the *DXS986* region, and preferably rs5968255. Another aspect of the present invention relates to a method for treating AIDS progression in a mammal based on said diagnosis. Other aspects include a method for screening for an anti-AIDS progression pharmaceutical compound, a pharmaceutical composition for the treatment of AIDS and/or AIDS progression, and a diagnostic kit.

### Background of the invention

The identification of host factors underlying differential progression to human AIDS is essential for the development of novel anti-retroviral therapies and novel vaccination concepts against HIV-1.

The risk for HIV-1 infection and the subsequent course of AIDS are substantially influenced by innate and adaptive immune defense proteins and by host factors that have a bearing on, for example, the entry of the virus and the viral life cycle (*1*). A functional genomic screen has identified more than 250 candidate mediators of the HIV-1 life cycle (*2*). In addition, genome-wide association studies (GWAS) of HIV-1 infected patients of European descent have unraveled a number of single nucleotide polymorphisms (SNPs) in the *MHC* region to be associated with viral load variation and differential disease progression (*3*). However, many more, hitherto unknown host factors are suspected to be involved the extremely variable progression of AIDS, and the type and nature of these factors may even depend upon ethnic backgrounds (*4*, *5*). Whereas the differences between males and females after HIV-1 infection for pharmacodynamics of anti-retroviral treatment are becoming aware, the consequences of genetic differences attributable to the X-chromosome are yet unknown, albeit they are suspected to underlie general immune response differences between the sexes (*6, 7*). This adds complexity to the impact of host factor variation on disease progression, antiretroviral treatment and vaccine efficacy.

It is therefore an object of the present invention, to provide additional genetic markers in order to further improve the diagnosis and/or treatment, and in particular the gender-specific diagnosis and/or treatment after HIV-1 infection, in particular with respect to a pharmacodynamics of anti-retroviral treatment.

### Summary of the invention

The present invention solves the above object through by providing a method, preferably an in vitro method, for diagnosing the likelihood and/or speed of AIDS progression in a mammal, comprising the analysis of at least one of the X-chromosomal SNPs from the *DXS986* region as defined by the SNPs rs825541 and rs2092545 in a sample obtained from said mammal.

Studies in the context of the present invention in the form of a microsatellite-based genome scan in the rhesus model of AIDS identified variation in the monkey *MHC* region and at a hitherto unknown locus on chromosome X as determinants of disease progression. Scrutinizing the X-linked finding for relevance in humans by reassessment of human genotypes in the orthologous region Xq21.1 from a recent genome-wide association study led to the identification of an intergenic SNP, as being associated with AIDS progression. Heterozygous females showed a significantly slower drop of the CD4 T⁺ cells critical for antiretroviral treatment initiation, than homozygous females or hemizygote males. Furthermore, association analysis between rs5968255 and the viral load at set point (VL) yielded similar results. Here, CT heterozygous females showed a lower average VL than TT homozygous females (*P*=0.001), and no significant difference was again seen between T and C hemizygous males (*P*=0.2). An artifact due to a hidden association between rs5968255 and one of markers known to influence VL and AIDS progression in the CHAVI samples *(3),* rs2395029 (a proxy for *HCP5-HLAB*5701*) and rs9261174 (*ZNRD1*/*RNF39*), could be excluded. The SNP is located close to the *RPS6KA6* gene belonging to a family of ribosomal kinases that are involved in major signaling pathways for cell survival and cell proliferation and have been implicated in the growth-factor- and stress-induced activation of transcription factors. The inventors' results provide, for the first time, firm evidence that gender-specific differences in AIDS progression have a genetic basis as has long been suspected.

Preferred is the method according to the present invention, wherein said mammal is a mouse (preferably a respective mouse model) or human.

More preferred is the method according to the present invention, wherein said SNPs are selected from the SNPs rs825541, rs1531868, rs5968255, rs10218349, and/or rs2092545, preferably rs5968255.

More preferred is the method according to the present invention, wherein said mammal is a women, as it was surprisingly found that the C allele in Caucasian women was found as protective. As the C allele occurs 4 times more frequent in the Asian female population further preferred is the method according to the present invention, wherein said woman is an Asian woman, further preferred a woman from China or Japan.

According to another aspect of the present invention, a combination of SNP alleles is detected, consisting of 2, 3, 4, or 5 SNP alleles per locus (in one gene) as described herein, for example in Table 4. This combination allows for an additional improvement in accuracy during the diagnosis. Preferred is a method according to the invention, wherein the r² of the SNP in relation to the SNP rs5968255 is higher than 0.4, preferably higher than 0.8, and most preferred higher than 0.95.

Most preferably, the combination of SNPs provides for a detection with a statistical significance of an asymptotic *P* value < 0.05, preferably *P* < 0.005, more preferably *P* < 0.001, with respect to the diagnosis of the AIDS-progression relevant parameters as described herein, and/or contributions of individual SNPs to these AIDS-progression relevant parameter(s). In addition, a combination of the SNPs according to the present invention together with other statistically significant SNPs can be analysed. Such SNPs and respective haplotypes can be readily taken from the respective literature and included in the present set.

More preferred is the method according to the present invention, further comprising analysis of covariates, such as PC (principal components), such as PC2 and/or PC6. The additional analysis of PCs can further improve the relevance of the analyses according to the present invention. Another alternative is the additional analysis of expression markers, such as described below.

Preferred is the method according to the present invention, wherein the presence of the rs5968255 C allele indicates a significant slower decline of the CD4⁺ T cell counts and lower viral load in heterozygous HIV-1 infected females.

More preferred is the method according to the present invention, wherein said analysis is accomplished by sequencing, mini-sequencing, hybridisation, restriction fragment analysis, oligonucleotide ligation assay or allele specific PCR.

Applicable diagnostic techniques include, but are not limited to, DNA sequencing including mini-sequencing, primer extension, hybridisation with allele-specific oligonucleotides (ASO), oligonucleotide ligation assays (OLA), PCR using allele-specific primers (ARMS), dot blot analysis, flap probe cleavage approaches, restriction fragment length polymorphism (RFLP), kinetic PCR, and PCR-SSCP, fluorescent in situ hybridisation (FISH), pulsed field gel electrophoresis (PFGE) analysis, Southern blot analysis, single stranded conformation analysis (SSCA), denaturing gradient gel electrophoresis (DGGE), temperature gradient gel electrophoresis (TGGE), denaturing HPLC (DHPLC), and RNAse protection assays, all of which are presently known to the person skilled in the art and discussed in detail further below.

The presence of the disease predisposition due to the polymorphisms/variations at the altered sites (SNPs) in the *DXS986* region can be ascertained by testing any tissue of the human for those variations. The presence of such a polymorphism/variation can be determined by extracting DNA from any tissue of the body. For example, blood can be drawn and DNA extracted from blood cells and analysed. Moreover, prenatal diagnosis of the disease will be possible by testing foetal cells, placental cells or amniotic cells for polymorphism/variation in the locus/gene. There are several methods that allow the detection of specific alleles, and some of these methods are discussed here:

For a known polymorphism, direct determination of the respective genotype is usually the method of choice. State of the art approaches for high-throughput genotyping today rely on one of four different mechanisms: allele-specific primer extension, allele-specific hybridization, allele-specific oligonucleotide ligation and allele-specific cleavage of a flap probe (Kwok, Pharmacogenomics 1,95(2000)). Sequencing or mini-sequencing protocols are part of the primer extension methods, e.g. genomic DNA sequencing, either manual or by automated means can detect sequence variations in the *DXS986* region (Nucleic Acids Res 25,2032-2034(1997)). Minisequencing (primer extension) technology is based on determining the sequence at a specific base by allowing the elongation of a primer by one base directly at the variant site (Landegren et al., Genome Res. 8: 769-76 (1998)). Short sequence reactions coupled with an alternative detection method are the nature of real time pyrophosphate sequencing (Nyren et al., Science 281:363 (1998)).

Allele-specific hybridisation protocols rely on probes detecting one or several of the alleles present at the SNP positions. Several techniques were developed for detection of a hybridisation event. In the 5' nuclease assay and in the molecular beacon assay the hybridization probes are fluorescently labelled and probe binding is detected via changes in the behaviour of the fluorescent label (Livak, Genet. Anal. 14, 143 (1999); Tyagi et al., Nat. Biotechnol. 16, 49 (1998)). Hybridisation events may occur in liquid phase or with either the probe or the target bound to a solid surface. Hybridisation is thus also used when arrays (microchips) are used for genotyping purposes. This technique of nucleic acid analysis is also applicable to the present invention. A chip typically consists of thousands of distinct nucleotide probes which are built up in an array on a silicon chip. Nucleic acid to be analysed is fluorescently labelled (in the present case a sample containing the *DXS986* region or a portion thereof spanning the alterations) and hybridised to the probes on the chip. This method is one of parallel processing of thousands of probes at once and can tremendously accelerate the analysis. In several publications the use of this method is described (Hacia et al., Nature Genetics 14, 441 (1996); Shoemaker et al., Nature Genetics 14, 450 (1996); Chee et al., Science 274, 610 (1996); DeRisi et al., Nature Genetics 14, 457 (1996), Fan et al., Genome Res, 10, 853 (2000)).

Allele-specific oligonucleotide ligation assays have a high specificity. Oligonucleotides differing in the allele-specific base at the 5'- or 3'-end are only processed by a ligation reaction if they are perfectly bound to the template at the respective oligonucleotide end. This method has been coupled with fluorescence resonance energy transfer (FRET) labelling to create a homogeneous assay system (Chen et al. Genome Res. 8, 549 (1998)). Allele-specific cleavage of a flap probe uses the property of flap endonucleases (cleavases) to cleave structures created by two overlapping oligonucleotides. In this approach two overlapping oligonucleotides are bound to the polymorphic site. Which oligo has had a perfect match to the target sequence is then detected by the cleavage reaction (Lyamichev et al., Nat. Biotechnol. 17:292 (1999)).

Other methods which detect specific base variations usually allow a lower throughput only such as the allele-specific oligonucleotide (ASO) hybridisation. For allele-specific PCR, primers are used which hybridise at their 3' ends to one of the particular in the *DXS986* region base variations according to the invention. Only for alleles which are present, a respective PCR product is observed (Ruano and Kidd, Nucleic Acids Res 17, 8392 (1989)). A specificity increasing modification of allele-specific PCR is the Amplification Refractory Mutation System, as disclosed in European Patent Application Publication No. 0332435 and in Newton et al., Nucleic Acids Res 17, 2503 (1989). If the variations lead to changes in the specific recognition sites of nucleic acid processing enzymes methodologies such as restriction fragment length polymorphism (RFLP) probes or PCR-RFLP methods may also be used to detect these variations.

Other approaches detect only that changes with respect to a reference sequence are present in a nucleic acid. Many of these methods are based on the formation of mismatches when both SNP variants are present in the same sample. A popular method is the use of denaturing high performance liquid chromatography to separate heteroduplex from homoduplex molecules (DHPLC; Oefner, P.J. et al. Am J Hum Genet 57 (Suppl.), A266 (1995)). Another method is denaturing gradient gel electrophoresis (DGGE) (Wartell et al., Nucleic Acids Res 18, 2699, (1990); Sheffield et al., Proc Natl Acad Sci USA 86, 232 (1989)). By using DGGE, variations in the DNA can be detected by differential migration rates of allelic variants in a denaturing gradient gel. A variation is the clamped denaturing gel electrophoresis (CDGE; Sheffield et al., Am J Hum Genet 49, 699 (1991)), heteroduplex analysis (HA; White et al., Genomics 4, 560 (1992)) and chemical mismatch cleavage (CMC; Grompe et al. Proc Natl Acad Sci USA 86, 5888 (1989)). The use of proteins which recognise nucleotide mismatches, such as the *E. coli* mutS protein may help in detecting mismatched DNA molecules (Modrich, Ann. Rev. Genetics, 25, 229 (1991)). In the mutS assay, the protein binds only to sequences that contain a nucleotide mismatch in a heteroduplex between mutant and wild-type sequences. RNase protection assays are another option (Finkelstein et al., Genomics 7, 167 (1990)). The RNAse protection assay involves cleavage of the mutant fragment into two or more smaller fragments. Another way is to make use of the single-stranded conformation polymorphism assay (SSCP; Orita et al., Proc Natl Acad Sci USA 86, 2766 (1989)). Variations in the DNA sequence in the *DXS986* region from the reference sequences will be detected due to a shifted mobility of the corresponding DNA-fragments in SSCP gels. SSCP detects bands which migrate differently because the variation causes a difference in single strand, intra-molecular base pairing.

Indirect methods as described above for the detection of sequence variations would be particularly useful for screening relatives for the presence of a sequence variation found previously in an affected family member.

Other approaches for detecting small sequence variations as known for those skilled in the art can be used.

Detection of polymorphisms/point mutations may be accomplished by amplification, for instance by PCR, from genomic or cDNA and sequencing of the amplified nucleic acid or by molecular cloning of the *DXS986* region allele as defined by the SNPs rs825541 and rs2092545 and sequencing the allele using techniques well known in the art.

More preferred is the method according to the present invention, further comprising an analysis of the expression at least one of the proteins CYLC1 (cylicin 1), HDX (highly divergent homeobox), POU3F4 (POU class 3 homeobox 4) and RPS6KA6, preferably RPS6KA6 (ribosomal protein S6 kinase). Expression analysis can be performed using methods known to the person of skill, such as protein expression analyses, and/or gene expression analyses involving, for example, nucleic acid chip technologies, and/or antibodies. Preferred is an ELISA.

Another aspect of the present invention the relates to a method, preferably an in vitro method, for diagnosing the likelihood and/or speed of AIDS progression in a mammal, comprising the analysis of the expression or the biological function at least one of the proteins *CYLC1* (cylicin 1), HDX (highly divergent homeobox), POU3F4 (POU class 3 homeobox 4) and RPS6KA6, preferably RPS6KA6 (ribosomal protein S6 kinase) in a mammal.

Expression analysis can be performed using methods known to the person of skill, such as protein expression analyses, and/or gene expression analyses involving, for example, nucleic acid chip technologies, and/or antibodies. Preferred is an ELISA. It was surprisingly found that the expression of these markers and/or their biological function could be correlated with the likelihood and/or speed of AIDS progression in a mammal.

Preferred is the method according to the present invention, further comprising measuring the change or absence or presence of the biological function of the protein CYLC1 (for example the extent of spermiogenesis and/or the formation of the cytoskeleton), HDX (for example transcription control), *POU3F4* (for example transcription control) and/or *RPS6KA6* (for example the kinase activity and/or signal transduction), and/or the decline of the CD4⁺ T cell counts and immunodeficiency virus replication kinetics compared to a control sample or patient.

Another aspect of the present invention then relates to a kit comprising materials for analysing the expression of proteins CYLC1, HDX, POU3F4 and/or RPS6KA6, in particular for an antibody-mediated detection or the detection of a modification of said proteins, and/or for the analyses of the biological function of the proteins.

Another aspect of the present invention the relates to a method for treating AIDS progression in a mammal, comprising a method according to the present invention as above, and providing an anti-AIDS progression medical treatment, in particular a treatment against a decline of the CD4⁺ T cell counts and/or an anti-retroviral treatment to said mammal in need thereof. Respective treatments are known to the person of skill and described in the respective literature of the treatment of retroviral diseases and AIDS.

More preferred is the method according to the present invention, wherein said mammal is a women, as it was surprisingly found that the C allele in Caucasian women was found as protective. As the C allele occurs 4 times more frequent in the Asian female population further preferred is the method according to the present invention, wherein said woman is an Asian woman, further preferred a woman from China or Japan.

Another aspect of the present invention the relates to a method, preferably *in vitro,* for screening for an anti-AIDS progression pharmaceutical compound, comprising the steps of: a) measuring the expression of at least one of the proteins CYLC1, HDX, POU3F4 and RPS6KA6, preferably RPS6KA6, in a cells from a mammal having an immunodeficiency virus-infection, preferably an HIV-infection, or in experimentally immunodeficiency virus-infected cells from a mammal, b) contacting said protein *CYLC1, HDX, POU3F4* and/or *RPS6KA6,* with at least one potentially interacting compound, and c) measuring the change of the expression of the protein CYLC1, HDX, POU3F4 and/or RPS6KA6 in the presence of said compound from step b).

Preferred is the method according to the present invention, further comprising the steps of: d) measuring the biological function of the protein CYLC1 (for example the extent of spermiogenesis and/or the formation of the cytoskeleton), HDX (for example transcription control), *POU3F4* (for example transcription control) and/or *RPS6KA6* (for example the kinase activity and/or signal transduction), and/or the decline of the CD4⁺ T cell counts and immunodeficiency virus replication kinetics in the presence or absence of said binding compound.

More preferred is the method according to the present invention, further comprising the step of performing a diagnostic method according to the present invention as above.

More preferred is a method for producing a pharmaceutical composition for the treatment of AIDS and/or AIDS progression, comprising the method according to the present invention as above, and further comprising the step of admixing said compound changing the expression of the proteins of HDX, POU3F4, CYLC1 and/or RPS6KA6 with suitable auxiliary substances and/or additives.

Another aspect of the present invention the relates to a pharmaceutical composition for the treatment of AIDS and/or AIDS progression produced according to the present invention, comprising a binding compound isolated by the method according to the present invention and, optionally, suitable auxiliary substances and/or additives.

Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

Finally, yet another aspect of the present invention the relates to a d kit, in particular a diagnostic kit comprising materials for performing the method according to the present invention as above, in particular materials for analysing SNPs in a mammal that are selected from the SNPs rs825541, rs1531868, rs5968255, rs10218349, and/or rs2092545, and preferably rs5968255.

In a preferred embodiment, said kit further comprises materials for analysing the proteins CYLC1, HDX, POU3F4 and/or RPS6KA6, in particular for an antibody-mediated detection or the detection of a modification of said proteins.

The kit can contain other compounds such as enzymes, buffers, and/or dyes for performing the method(s) of the present invention. In another example, the kit according to the invention is suitable to perform a chip-based analysis in at least one SNPs in the *DXS986* region. The kit can also include instructions for performing the SNP-analysis and/or the software for a statistical analysis as described herein.

To identify novel host factors influencing the disease course after HIV-1 infection the inventors used the rhesus macaque model of AIDS. Despite some pathophysiological differences that distinguish simian immunodeficiency virus (SIV) infected non-human primates from HIV-1 infected humans, the rhesus macaques represent the most important model for pre-clinical proof-of-concept studies of HIV vaccines and microbicides as well as for pathogenicity studies. In addition, the rhesus model may serve as a means to identify host genes influencing vaccination efficacy (*8*). To improve the model, it is essential to determine which host factors impacting upon infection are shared by humans and rhesus macaques, and which are species-specific. Unfortunately, genetic screening technologies such as genome-wide SNP arrays and HapMap-like resources have not yet been developed for non-human primates.

The inventors performed a microsatellite-based, genome-wide association analysis in rhesus macaques of Indian descent (*Macaca mulatta*) that displayed differential progression to AIDS (*9*) (Table 2). The microsatellites used for the genotyping were almost all derived from the initial linkage map in rhesus monkeys (*10*) (Table 3). Originally, the inventors intended to pursue linkage mapping, based upon the family structure of the rhesus macaques kept at the German Primate Centre (DPZ). This approach was dismissed, however, since the number of certain patrilineal relationships would have been too small to achieve sufficient statistical power (data not shown). The inventors therefore adopted an association-based design using 136 SIV-infected monkeys as the screening samples to investigate the microsatellites for their potential association with disease progression. A second group of 128 SIV-infected rhesus macaques that were of various origins and unrelated to the animals of the screening samples was used for replication (Table 1). For a consistent phenotype definition, the inventors developed a scoring system for AIDS-free survival time in order to account for the fact that some monkeys were euthanized without AIDS-related symptoms or were still alive by the end of the present study. Since approximately 40 % of the monkeys were immunized prior to infection, the scoring system was also adjusted to account for such pre-treatment. Both, screening and replication samples were similar in terms of the average scoring and the average time of AIDS-free survival (Table 1).

A number of microsatellite markers were genotyped in the screening samples and achieved a call-rate >90%. The markers used had a nominal average spacing of about 9 centiMorgans (cM). In the association analysis two markers were found to be significantly associated with survival time after Bonferroni correction, namely *D6S2972* (Monte-Carlo Markov-Chain implementation of Fisher's exact test: *P*≤10⁻⁶) and *D6S2704* (*P*=1.2×10⁻⁴) (*9*). Both markers were located in the rhesus *MHC* (major histocompatibility complex) class I region, 400 kb apart from each other and flanking the A-region. *D6S2704* is located 150 kb distal to the *ZNRD1* and *RNF39* genes. Polymorphisms associated with both genes have been reported to influence AIDS progression in humans (*3*). *ZNRD1* was also identified in a recent functional genomic screen for HIV-1 dependency factors involved in the viral life cycle (*2*). Marker *D6S2972* is located 40 kb downstream of *Mamu-F*, the rhesus orthologue of *HLA-F*. The importance of the *MHC* for the protection against retroviral infection is known for both rhesus macaques and humans (*1, 11*) and also reflected by the fact that in addition to the two *MHC* markers that passed Bonferroni correction, further eight MHC-specific markers were among the top 50 nominal significant P-values in the inventors' association screen.

In order to validate the results in the screening cohort, six *MHC*-specific markers and 5% of markers representing loci on other chromosomes, including two X chromosomal loci with a *P*-value ≤0.005, were selected for replication. For each of the non-*MHC* loci, two additional flanking markers, ± 200-300 kb apart, were also included in the validation panel. Several markers were genotyped in the replication samples. Four *MHC* markers, including *D6S2704* and *D6S2972,* ranked among the markers with nominal significant *P*-values. The X-chromosomal markers *DXS986* (*P*=1.3×10⁻⁴) ranked third, and both its flanking markers (*DXS986*+*1, P*=1.0×10⁻⁶; *DXS986-2, P*=8.4×10⁻⁵) passed Bonferroni correction. As both, the screening as well as the replication samples were underpowered concerning female rhesus monkeys, the inventors were not able to detect a significant difference in the survival time between the two sexes. The underrepresentation of female rhesus monkeys in the inventors' study samples reflects the situation of primate centers having to breed animals and retain female monkeys to obtain offspring.

Sex-specific genetic factors have been suspected for some time to influence the course of AIDS in humans, but could not be established firmly by epidemiological studies so far (*6*, *7*). The inventors therefore investigated whether the rhesus X-chromosomal association is relevant for human HIV-1 infected patients. To this end, the inventors analyzed disease progression data and X-chromosomal single nucleotide polymorphism (SNP) markers from a recent genome-wide association scan performed by the Center for HIV/AIDS Vaccine Immunology (CHAVI) (*3*). The inventors used data from 486 samples stratified by sex (119 females, 367 males) for which the progression to AIDS was measured by the time until the CD4⁺ T cell count had dropped to the value of 350 cells µL⁻¹, which is critical for initiation of antiretroviral treatment (ART). After confirming synteny between the region surrounding *DXS986* in rhesus and in the orthologous Xq21.1 region of humans (*9, 12*), the inventors performed an independent association analyses of the human progression phenotype in that region (NCBI Build 36.1 map coordinate: 73,657,227-90,030,939) . Out of the X-chromosomal 13821 SNPs, 145 SNPs were located within that selection. Progression scores were normalized by taking the natural logarithm. To account for possible population stratification, the inventors calculated the first six principal components (PC) for all X-chromosomal markers using EIGENSTRAT and considered those in the subsequent analysis which showed a significant association with progression. Nine individuals were excluded by EIGENSTRAT for being genetic outliers, leaving 477 individuals (118 females, 359 males) for subsequent analysis. Out of these individuals, 174 had missing progression scores. In total, 93 females and 210 males had complete information on progression and on principal components. Upon Bonferroni correction, single-marker ANOVA without PC incorporation revealed six SNPs from the *DXS986* region as being significantly associated with AIDS progression. If PCs were included as covariates, only the association with rs5968255 remained significant. Notably, no significant association was observed amongst the males. The inventors next performed a multiple linear regression analysis of the six SNPs that were found to be significantly associated with the progression phenotype of the females without PC incorporation. Backward AIC-based model selection with PC incorporation confirmed SNP rs5968255 as being the only significantly associated marker after Bonferroni correction (*P*=1.3×10⁻²) in the *DXS986* region (nominal *P*=8.8×10⁻⁵ in females, *P*=0.19 in males). A relaxed multiple-testing correction did not alter the association results.

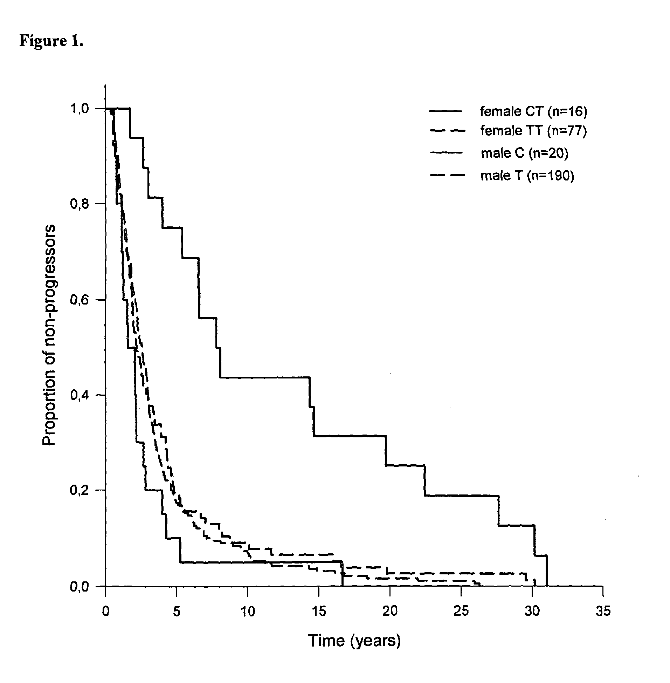

Marker rs5968255 is located within a 500 bp conserved intergenic region 3.9 Mb distal to *DXS986,* 64 kb proximally to the ribosomal protein S6 kinase 6 (*RPS6KA6;* alias *RSK4*) gene and 138 kb distally to the cylicin 1 gene (CYLC1; basic protein of sperm head cytoskeleton; Fig. 1). The *RPS6KA6* (*RSK4*) encodes a gene product belonging to a family of four ribosomal serine/threonine kinases (RSK1-4), of which the three that have been studied in detail (RSK1-3) are involved in MAPK-, ERK- and mTor-signaling important for cell proliferation and survival (*13*). All four proteins may function as mediator of the growth-factor- and stress-induced activation of transcription factors. For example, RSK2 can regulate cAMP regulatory element binding protein (CREB) which interacts with HIV-1 protein Tat and is required for the life cycle for HIV-1 (*14*). All four proteins (RSK1-4) may function as mediators of the growth-factor- and stress-induced activation of transcription factors. Consequently, they have been examined in detail mainly in cancer studies but, at least indirectly, these proteins may also be involved in HIV-1 infection. RPS6KA6 may be different from the other ribosomal kinases in that is seems to be fully activated in unstimulated cells (32) and over-expression in human breast cancer cells results in suppressed proliferative activity of these cells (33). Another indirect link with HIV-induced disease may derive from the fact that over-expression of RPS6KA6 results in up-regulation of Claudin-2 and down-regulation of the chemokine receptor CXCR4. CXCR4 is highly expressed in human malignant breast tumours and is a co-receptor of HIV-1. Furthermore, RPS6KA6 may be regulated by c-Myc which is also involved in HIV-1-expression (34). So far, it is not known whether RPS6KA6 is involved in CXCR4 receptor expression in lymphocytes, but it may be speculated that CXCR4 expression is related to progression to AIDS. HIV-1 transmission usually occurs by infection with viruses utilizing the chemokine receptor CCR5 for entry. However, in the course of the AIDS, HIV-1 variants arise that can use CXCR4 as a co-receptor. Although, the underlying mechanism is still enigmatic, this virus-co-receptor switch is known to be associated with progression to AIDS (35). Claudin 2 is a tight-junction protein that is expressed, for example, in the intestine, i.e. a tissue that is severely impaired by HIV-infection. Claudin proteins have been implicated in virus entry and dissemination in various infections, including HIV-infection of the brain (37-39). Notably, Claudin 2 is also encoded by an X chromosomal gene (at Xq22.3).

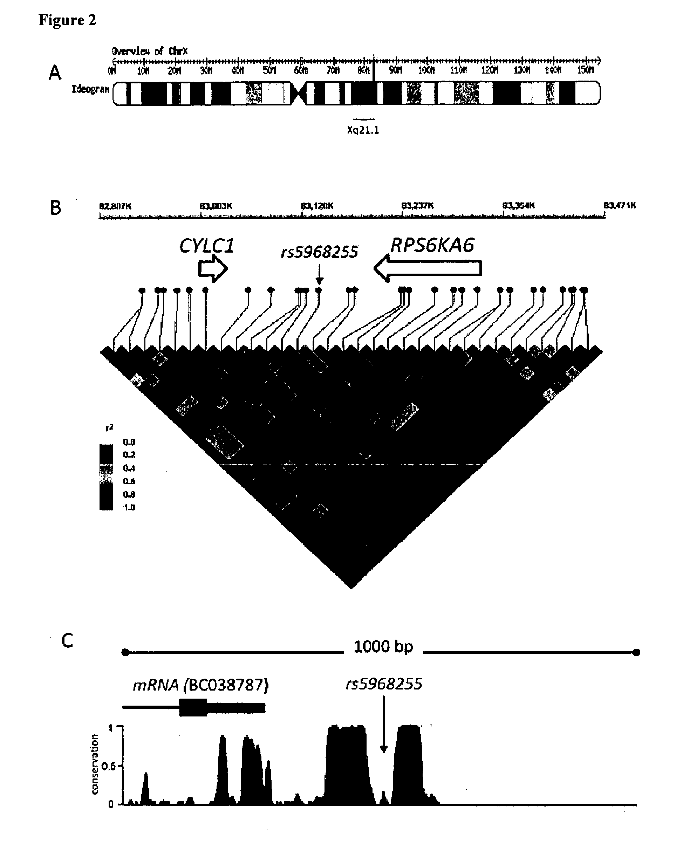

The rs5968255 allele and genotype frequencies found in the CHAVI samples were fully consistent with data for HapMap CEU (Utah residents with ancestry from northern and western Europe; Caucasians; Re123a; NCBI 36; dbSNP b126) showing C as the minor (0.125) and T as the major allele (0.875). Using chimpanzee as outgroup, the T allele is believed to represent the ancestral state. When the inventors' samples were stratified for the sexes, 90.5% of the males were hemizygous for the rs5968255 T allele, while 9.5% where hemizygous for the C allele. Of female samples, 83 % were from TT homozygous carriers and the remaining 17 % were TC heterozygous. No CC homozygotes were observed. As shown by Kaplan-Meier-analysis of the progression time to ART initiation in Fig. 2, the rs5968255 C allele conferred a significant slower decline of the CD4⁺ T cell counts to heterozygous HIV- 1 infected females than to women homozygous for the T allele (*P*=0.001). In contrast, neither the C nor the T allele did convey any apparent advantage to hemizygous males (Fig. 1). Additionally, heterozygous females showed higher log-progression scores (8.10±0.92, IQR 7.51-8.91) than homozygotes (6.82±0.96, IQR 6.17-7.38). No such trend was apparent between male hemizygotes (T: 6.81±0.85, IQR 6.25-7.27; C: 6.52±0.80, IQR 6.02-6.90). In the linear regression analysis, SNP rs5968255 explained between *R²*=21% (no adjustment for population stratification) and *R²*=25% (including of PC2 and PC6 for population stratification adjustment) of the log-progression score variation observed in females in the inventors' present invention. Association analysis between rs5968255 and the viral load at set point (VL) yielded similar results (Table 4; Table S8). Here, CT heterozygous females showed a lower average VL than TT homozygous females (*P*=0.001), and no significant difference was again seen between T and C hemizygous males (*P*=0.2). An artifact due to a hidden association between rs5968255 and one of markers known to influence VL and AIDS progression in the CHAVI samples, rs2395029 (a proxy for *HCP5-HLAB*5701*) and rs9261174 (*ZNRD1*/*RNF39*), could be excluded. The polymorphism displays a clear visible influence for heterozygotes. However, the majority of HIV-1 infected women of European descent (83%) progresses with the same rate as the male patients into the potentially symptomatic phase, requiring ART to ameliorate their CD4⁺ T cell status again. Comparison of the rs5968255 allele and genotype frequencies in the four HapMap populations showed that the advantageous allele is even less frequent in the Sub-Saharan-Africans (0.025) than in Caucasians (0.125), while it is quite prevalent in the Asian population of Chinese (0.467) and Japanese (0.58) origin (Table 4). The inventors' analysis suggests that many more Asian HIV-1 infected females heterozygous at rs5968255 experience a longer asymptomatic period after HIV-1 infection than Caucasians, and even longer than Sub Saharan Africans. To follow up the inventors' findings it would be extremely helpful to study Asian AIDS patients because many more rs5968255 CC homozygotes and C hemizygotes can be expected in these populations than in Caucasians or Africans.

Since rs5968255 is not necessarily causal for the observed association the inventors next identified those SNPs in the *DX6986* region that showed at least moderate LD (***r**²*≥0.4) with this marker in the HapMap's CEU (Caucasians) samples (*15, 16*).

After HIV-1 infection, the number of CD4⁺ T cells µL⁻¹ in women tends to be higher and correlates with lower viral RNA levels than in men (*6*, *7*). Yet, it is still controversial whether this contributes to a significant difference in progression to AIDS between males and females, and whether there are population-specific differences (*6*, *7*). The possible consequences of a generally higher level of CD4⁺ T cells in females has spurred discussions about changing the guidelines for treatment initiation in females (*7*). The inventors' findings indicate that only a minor proportion (17%) of infected Caucasian females may experience a prolongation of their asymptomatic stage through a slower depletion of CD4⁺ T cells genetically linked to the rs5968255 C allele in contrast to the majority of females (83%), who eventually progress as rapid as males into the symptomatic state of AIDS. On the other hand, the inventors' results may reflect more general sex differences in the human immune response that are not specific for HIV-1 infection alone. Since it is known that the higher CD4:CD8 ratio in females compared to males is genetically determined (*17*), with the underlying genetic variation(s) yet to be determined, the inventors' results may represent a very first step towards disclosing the genetic basis of such differences.

Apparently, there are still gaps in the inventors' understanding of immune responses of both sexes and how HIV-1 affects the sexes differently (*6*, *7*). The fact that an estimated 6% of HIV-1 dependency factors (HDF) necessary for the life cycle of the virus may be X-linked (*2*) suggests that there is much more genetic variation than uncovered by the inventors' present invention which is likely to play a role in the sex-specificity of HIV-1 infection and AIDS. These differences may affect therapy protocols or vaccination efficacy.

The invention will now be further described based on the following examples and with reference to the accompanying figures and the sequence protocol, without being limited thereto. For the purposes of the present invention, all references as cited herein are incorporated by reference in their entireties. In the Figures,
Figure 1 shows the Kaplan-Meier analysis of AIDS progression, stratified by rs5968255 genotype and sex. The horizontal axis measures the time until the individual CD4⁺ T cell count has dropped to 350 cells µL⁻¹, i.e. the recommended threshold for antiviral treatment initiation after HIV-1 infection. In females, carrying the C allele of rs5968255 significantly delays the time to antiviral treatment initiation (*P* = <0.001).
Figure 2 shows a part of the region flanked by the 6 SNPs according to the present invention. (A) Ideogram of chromosome X. The position of rs5968255 at Xq21.1 (NCBI Build 36.1: 83,141,348) is indicated in red. (B) Partial map of Xq21.1 (82,887,000-83,471,000) depicting the location of genes close to rs5968255 (marked by an arrow) and the local pattern of linkage disequilibrium in the HapMap's CEU sample. This graph was created with the WGAViewer (29). (C) Evolutionary conservation within a 1 kb window centered around rs5968255 (83,140,849-83,141,848); the conservation data were obtained from the UCSC Genome Browser.

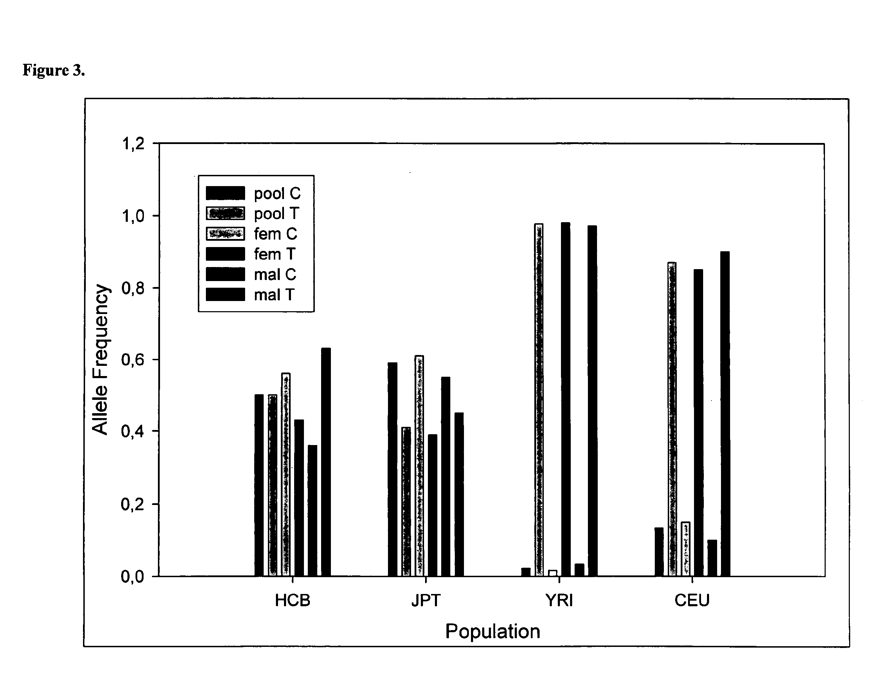

Figure 3 shows the allele frequencies of marker rs5968255 in HapMap. Frequencies differ significantly between CEU and YRI, respectively, versus the Asian populations CHB and JPT. While the frequencies of the minor C allele and the major T allele are somewhat similar for the populations of European and African descent in both females (fem) and males (mal), they both differ from those of the Asian populations which are also differing among themselves. The pool data summarize allele frequencies irrespective of sex.

### EXAMPLES

### Description of monkey samples

All SIV-infected animals were housed at the DPZ according to the German Animal Protection Act, which complies with EU guidelines on the use of non-human primates for biomedical research. The rhesus monkeys analyzed in the present invention were of Indian descent and have been used in several experimental studies before (Table 1 and Table 2). For all monkeys, the time point and route of SIV infection, the date of death, previous treatments, dose of infecting virus as well as the infecting virus isolate itself are known. Necropsy results, regular hemograms and antibody titers are available for all monkeys. Cell-associated viral load data and viral RNA copy number are known for the majority of animals. Most monkeys were infected with SIVmac251 or SIVmac239. The type of infecting virus has had no statistically significant influence upon survival time (*20*).

### Definition of AIDS-related disease

Monkeys that were euthanized with signs of AIDS suffered from anorexia, incurable diarrhea, *Pneumocystis carinii* infection or neurological dysfunction as evidenced by ataxia. Diagnoses were based upon clinical, necropsy or post mortem immunochemistry CD4⁺ T cell counts were found to be an inadequate marker for the diagnosis of AIDS since many rapid progressors still had increasing CD4⁺ counts by the time of euthanasia (*18*, *21*).

### AIDS progression score

46% of the monkeys from screening cohort and 22% from replication cohort were euthanized before they developed AIDS-related symptoms or are still alive. Therefore, survival time appeared to be inappropriate to estimate disease progression. In addition, about 40% of the monkeys were immunized with a prospective AIDS vaccine before infection. None of the vaccines tested were able to reduce set-point viral load significantly. However, the vaccination may have prolonged survival time (Table 1). To overcome these caveats, disease progression of the monkeys were scored by 5 points (Table 3). For scoring, the necropsy results, antibody titer, available viral load data, and neopterin values of each animal were inspected manually. To score those monkeys which did not die with AIDS-related symptoms and did not pass the survival time necessary to be scored with 4 (19 monkeys from the screening cohort including 2 elite controllers, 7 monkeys from the replication cohort), these parameters were additionally used to score disease progression.

Monkeys with a cell-associated set-point viral load >0.1 infected cell per 10⁶ cells or RNA plasma load below 1000 RNA copies mL⁻¹ plasma, showing no signs of immunodeficiency (as determined by the CD4 counts) or inflammatory reactions (necropsy results) were scored with 5 points as elite controllers. Of the 12 elite controllers from the DPZ cohort 10 were observed for a period of at least 120 weeks post infection. The two other animals scored as elite controllers presented with very low to undetectable viral loads after immunization. Of the 9 elite controllers from the replication cohort, only one monkey with undetectable viral loads was studied for less than two years.

However, the immunized macaques had a higher score than the non-immunized macaques, partially because there were more elite controllers among the immunized macaques (Table 1), and because - most pronounced in the DPZ cohort - potential rapid progressors were withdrawn from vaccine studies (*18*). However, closer inspection of the elite controllers revealed that among the DPZ animals, the majority were from a newly formed breeding group in which the elite controller phenotype was obviously inherited. Similarly, the percentages of elite controllers was highest among the monkeys originating from the Defence Science and Technology Laboratory (DSTL, GB) irrespective of their pre-treatment (12.5 %). Since the majority of the monkeys from DSTL were immunized, this influenced positively all score of the immunized monkeys. Therefore, the inventors concluded that the risk for confounding effects of immunization was negligible in this present invention.

### Microsatellite-based genotyping

The inventors conducted a genome-wide analysis in 136 SIV-infected rhesus monkeys as the screening samples (*Macaca mulatta*) using initially a set of microsatellite markers (∼ 8 centi-Morgan resolution. Human as well as *Macaca mulatta*-specific microsatellites were used that were mainly based on marker information of Jeff Rogers (*10*). To further genotype interesting non-*MHC* loci the inventors designed two flanking markers per locus (in 200-300 kb distance up- and downstream, respectively) and 3 primer pairs located at the killer-immunoglobulin-like receptor (KIR) region. To define these markers, the inventors conducted a whole-genome screen for microsatellite sequences using a Perl script applied to the *Macacca mulatta* genomic sequence published by NCBI (http://www.ncbi.nlm.nih.gov). Assay design for appropriate markers and for standard optimization procedures was done by using PRIMER 3 (http://frodo.wi.mit.edu/). Markers (comprising initial non-*MHC* markers, flanking marker, and 6 selected *MHC* markers and 3 KIR markers) were genotyped in the replication samples. Furthermore, the screening samples were re-screened with the flanking primers and the 3 KIR markers. The inventors' microsatellite-platform measures fragment-sizes by separating PCR products with an ABI 3700 capillary sequencer (Applied Biosystems), and subsequently analyses them with Genemarker software (Softgenetics LLC).

### Statistical analysis

### Microsatellite-based association analysis in screening and replication samples

In the screening and replication samples, 136 and 128 SIV-infected animals were typed for 351 and 84 microsatellite markers, respectively. All markers had a call rate > 90 %. Hemizygosity of X-chromosomal markers was verified in males, and were otherwise excluded from the association analysis. In addition, the heterozygosity rate, and the portion of father-mother-infant trios with Mendelian errors were calculated as process control parameters and lead to a reduction of the panel size to fewer markers. There was no association between call rate and Mendelian error rate. To obtain most conservative values, no marker was disregarded for calculating Bonferroni correction. Allelic association with the AIDS progression scores (Table 3) was tested for each microsatellite using Fisher's exact Test. The P-values were calculated by Monte-Carlo Markov-Chain simulation. Bonferroni correction for multiple testing was carried out in the screening samples. In the replication samples, each original marker and its two flanking markers, were treated as a marker group. Then, the P-values were first corrected according to Holm within each of the 26 groups, and the most significant marker selected to represent the marker group. In the second step, the P-values of the group representative and of six additional singular markers were again corrected for multiple testing according to Holm.

### Data and quality control for SNP-based association analysis in humans.

The inventors analyzed the genotypes of 13821 X-chromosomal SNPs, determined in 486 samples (119 females, 367 males) and kindly provided to us (*3*). Genotyping was carried out using Illumina HumanHap550 Bead arrays, followed by ad-hoc quality control as described (*3*). Correct sex assignment was checked using the proportion of heterozygous genotypes at all markers (males: <1%, females: >26%). Average pair-wise identity-by-state (IBS) was used to assess extreme genetic similarity and dissimilarity, pointing at cognate relatives or genetic outliers, respectively. For post-hoc quality control after association analysis (see below), the inventors required the nominally significant SNPs to have a call-rate of more than 95% and obey Hardy-Weinberg equilibrium at the 0.05 level, using an exact test (*22*). Quality control was carried out with the R software version 2.6.2 (*23*).

### Assessment of population stratification.

To account for possible population stratification, the inventors used the principal components (PC)-based approach as implemented in EIGENSTRAT(*24*). Since EIGENSTRAT can only handle autosomal genotype data, the inventors assumed each male to be homozygous for all markers, while each female was doubled in the EIGENSTRAT analysis. A recent survey of genome-wide autosomal genetic variation in Europe (*25*) showed considerable variation only in the first five to six PCs of autosomal markers. By analogy, the inventors therefore considered the first six PCs of the X-chromosomal markers for adjusting for potential population stratification.

### Association analysis.

Progression scores were normalized by taking natural logarithms. Deviation from normality was tested using a Shapiro-Wilk test. The inventors used ANOVA to test for association separately in females and males. The rare marker allele was assumed to act under an additive model in females. To take possible population stratification into account, the inventors subsequently used AIC-based backward selection in a linear regression analysis of the log- progression score and the six PC from the EIGENSTRAT analysis. Only PC2 and PC6 were retained and were subsequently included in the association analysis. Multiple-testing correction was applied separately for the two X-chromosomal regions under study. All analyses were carried out using R 2.6.2 (*26*).

### Linkage disequilibrium analysis.

The inventors used HaploView 4.1 (*27*) to quantify linkage disequilibrium (LD) in the sample sets and to define tagSNPs (*28*), separately for male and female samples. The inventors used the CEU (Utah residents with ancestry from northern and western Europe) genotype data of the HapMap project (*15, 16*), release 23a (phase II, March 2008, http://ftp.hapmap.org/genotypes/2008-03/forward/non-redundant/) to infer which SNPs were in LD at the population level.

### Kaplan-Meier survival analysis.

The inventors used the Kaplan-Meier survival/log rank procedure to assess the statistical significance of progression time differences in the CHAVI samples. Kaplan-Meier graphs were created in R (*23*) version 2.6.2 and Sigmaplot 11 (Systat Software, Inc.)

### Synteny analysis

Synteny between the X-chromosomal regions in humans and macaques was checked using the tuple_plot program for pair-wise nucleotide sequence comparison with noise suppression (*12*). The thuman Xq21.1-chromosomal region spanning coordinates 73,657,227-90,030,939 (human assembly, NCBI Build 36.1 at http://genome.ucsc.edu) were compared to the X-chromosomal rhesus region spanning 73,657,227-90,030,939 (*Macaca mulatta* assembly, Mmul_051212 from the Baylor College of Medicine Human Genome Sequencing Center at http://genome.ucsc.edu).

### Association analysis and listing of all marker P-values for genotyping the screening and replication samples

Microsatellite markers were genotyped in the screening samples requiring a call-rate >90%. In the association analysis (Monte-Carlo Markov-Chain implementation of Fisher's exact test) with the screening samples (Table 3) four markers were found to be significantly associated with survival time according to the inventors' scoring system (Table 3) after Bonferroni correction, namely *D6S2972* (*P*≤10⁻⁶), *D6S2704* (*P*=1.2×10⁻⁴; *D2S434 (P*=2.4×10⁻⁵) and *MML9S30* (*P*=0.1×10⁻⁵). A discussion of the markers *D2S434* and *MML9S30* was disregarded, because they were badly replicated, e.g. *D2S434* being one of the last ranking markers in the replication samples (see Table 3).

### Association of D6S2972 and D6S2704 alleles with survival in screening and replication samples

In the DPZ screening samples only 4 alleles of *D6S2972* were detected. Two of them were by 100 % linked to the *MHC* class I alleles *Mamu-A*01* known to be associated with slow disease progression in SIV-infected rhesus macaques (34 A*01 positive of 34 monkeys carrying one of these *D6S2972* alleles *P*=0.00001,χ² test) (*11, 30, 31*). This linkage may explain the association of *D6S2972* with survival time in the DPZ monkeys. In contrast, in the replication samples marker *D6S2972* did not pass the Bonferroni test probably because the alleles of *D6S2972* were less tightly linked to *Mamu-A*01* compared to the screening sample. However, *Mamu-A*01* was in both samples significantly associated with survival time even when immunized and naïve-infected monkeys were analyzed separately (*P*< 0.002, Kaplan-Meier survival analysis).

The association of *D6S2704* with survival can probably also partly be explained by its association with particular *MHC* genotypes within the DPZ colony. The causal variation underlying this association will remain elusive until a more detailed analysis of the non-*MHC* genes within this region including *ZNRD1* has been carried out.

### Quality control and accounting for population stratification in targeted association analysis in the human patient samples

The inventors applied post-hoc quality control measures as described in steps 5-7 (*3*). One of the SNPs had a call-rate <95% and was therefore excluded. The other five SNP markers (rs825541, rs1531868, rs5968255, rs10218349, rs2092545) obeyed Hardy-Weinberg law at the 0.05 level.

Principal components (PC) analysis is an established approach to account for possible population stratification in association analysis. The inventors analyzed all X-chromosomal markers using EIGENSTRAT (*24*). Since this software can only handle autosomal markers, the inventors assumed each male to be homozygous for all genotypes, while females were represented twice in the data set. The first six PC were subsequently subjected to AIC-based backward model selection in a linear regression model of the logarithmized progression scores. Only PCs 2 and 6 were kept in the model as yielding significant information. Sample-specific values of these two PC were then subsequently incorporated as covariates in the ANOVA-based association analysis.

### Relaxed multiple-testing correction

Some markers showed considerable allelic association with each other, rendering Bonferroni correction for multiple testing too strict of a method. The inventors therefore assumed the number of tagSNPs (*28*) to be a rough approximation of the number of independent hypotheses tested in each of the two regions under consideration. Pairwise SNP tagging was carried out using HaploView 4.1 (*27*). The inventors employed 0.8 and 0.5 as tagging thresholds, respectively, yielding 221 and 186 tagSNPs (out of 229 SNPs) in the *MMLXS21* region and 126 and 107 tagSNPs (out of 145 SNPs) in the *DXS986* region, respectively.

### rs5968255 allele and genotype frequencies in HapMap CEU

The HapMap (www.hapmap.org) is a database of common genetic variants in human beings. It describes these variants, and how they are distributed among people within populations and among populations in different parts of the world. Following populations are studied, YRI: Yoruba in Ibadan, Nigeria; JPT: Japanese in Tokyo, Japan; CHB: Han Chinese in Beijing, China, CEU: CEPH (Utah residents with ancestry from northern and western Europe).

C represented the minor rs5968255 allele amongst females in the HapMap populations CEU (0.15) and in YRI (0.0167). In contrast, the C allele occurred 4 times more frequent in the Asian populations CHB (0.56) and JPT (0.61). Like in the HIV-1 infected patients of the CHAVI cohort, no C homozygous genotypes were detected amongst females of CEU and YRI, respectively, whereas they can be fairly well observed amongst CHB (CC, 0.35; CT, 0.43) and JPT (CC, 0.41; CT, 0.41). In accordance with the inventors' findings, the hemizygous C allele carriers were also substantially underrepresented amongst CEU (0.1) and YRI (0.03), but are more or less equally present amongst males of Asian origin (CHB: 0.36; JPT: 0.54). Overall, the genotype and allelic differences between CEU females and males (except between males of CEU and YRI) and the other populations are statistically significant, whereas differences observed between the two Asian populations are not (Fisher's exact test). The differences are visualized (Fig. 3). The corresponding P-values are compiled below (Table 5).

### Survival analysis of progression time, stratified by rs5968255 genotype and sex

The log rank statistic for the progression curves in Figure 1 is greater than would be expected by chance; there is a statistically significant over all difference between the curves (*P*=<0.001). To isolate the group or groups that differ from the others the Holm-Sidak method was used for all pairwise multiple comparison procedures with an overall significance level of 0.05 (calculated with Sigmaplot version 11, Systat Software, Inc.):

| **Comparisons** | **Statistic** | **Unadjusted P Value** | **Critical Level** | **Significant** |
|---|---|---|---|---|
| male T vs. female CT | 21.465 | 0.00000360 | 0.00851 | Yes |
| female CT vs. male C | 19.184 | 0.0000119 | 0.0102 | Yes |
| female TT vs. female CT | 13.036 | 0.000306 | 0.0127 | Yes |
| male T vs. male C | 2.390 | 0.122 | 0.0170 | No |
| female TT vs. male C | 2.177 | 0.140 | 0.0253 | No |
| female TT vs. male T | 0.412 | 0.521 | 0.0500 | No |

Statistical analysis of the raw data for progression in the CHAVI cohort implies already a sex specific difference in progression to antiretroviral treatment initiation. Here, all CD4⁺ T-cell decline data to the threshold of ∼350 cells µL⁻¹ were distinguished only according to sexes (males or females). The log rank statistic for the survival curves is greater than would be expected by chance; there is a statistically significant difference between survival curves (*P* = 0.004).

**Table 1. SIV-infected rhesus monkeys in the screening and replication samples**

| | **Screening (N=136)** | **Replication (N=128)** |
|---|---|---|
| **Sex** | 101 males (74 %) | 110 males (86%) |
| | 35 females (26 %) | 18 females (14 %) |
| | | |
| **Average progression score** | | |
| All infected | 3.15 | 3.18 |
| - males | 3.16 | 3.19 |
| - females | 3.11 | 3.11 |
| - Naïve | 2.79 | 2.94 |
| - Pretreated | 3.71 | 3.52 |

**Table 2. SIV-infected rhesus monkeys (Macaca mulatta) used in the present invention^{a}**

| | **Screening samples (N=136)** | **Replication samples (N=128)** |
|---|---|---|
| **Origin of monkeys** | all from DPZ | 19 from DPZ |
| | | 62 from Morgan Island Colony, US |
| | | 32 from DSTL, GB |
| | | 15 from CPRC, Puerto Rico |
| **Status before infection** | | |
| Naïve | 75 (55%) | 79 (62%) |
| Immunized | 54 (40 %) | 48 (37%) |
| Short term antiviral treatment | 7 (5%) | 1 |
| **Infecting virus** | | |
| SIVmac239 | 54 (40%) | 56 (43.7%) |
| SIVmac251 | 38 (28%) | 51 (39.8%) |
| SIVmac251/32H | 33 (24%) | 15 (11.8%) |
| SIVmac251/32H/*ex* vivo isolate | 11 (8%) | 1 (0.8 %) |
| SIVmac239/I324D +334PL | None | 3 (2.3 %) |
| SIVmac239, nef-stop | None | 2 (1.6 %) |
| **Route of infection** | | |
| Intravenously | 86 (63%) | 82 (64%) |
| Tonsillary | 37 (27%) | 35 (27%) |
| Intrarectally | 13 (10%) | 11 (9%) |
| | | |
| Number of naïve infected elite controllers | 3 of 54 macaques (5.5%) | 2 of 78 macaques (2.6%) |
| Number of elite controllers after immunization | 10 of 55 macaques (18%) | 6 of 48 monkeys (12.5%) |

| | | |
|---|---|---|
| **^{a}, Abbreviations** | | |

DPZ: German Primate Center; DSTL: Defence Science and Technology Laboratory, CPRC: Caribbean Primate Research Center. The rhesus macaques of the DPZ originate from the Caribbean Primate Research Center (CPRC), Puerto Rico, Morgan Island Colony, USA, and the Centre de Primatologie, France.

**Table 3 AIDS progression scoring system**

| Treatment: | None | Immunization |
|---|---|---|
| score | Survival time | Survival time |
| | (wpi) | (wpi) |
| 1 | 0-14 | 0-20 |
| 2 | 16-32 | 21- 40 |
| 3 | 33-56 | 41-60 |
| 4 | >56 | >60 |
| 5 | Elite controller | Elite controller |

**Table 4. Markers with at least moderate allelic correlation with marker rs5968255 in HapMap CEU**

| **SNP** | **Position** | **r²** |
|---|---|---|
| rs825541 | 82936504 | See Text |
| rs825552 | 82875071 | 0.90 |
| rs1095322 | 82883040 | 0.90 |
| rs1531868 | 82911428 | 0.90 |
| rs12559550 | 82967614 | 0.40 |
| rs1576904 | 82981996 | 1.00 |
| rs5968247 | 82984598 | 1.00 |
| rs2151168 | 82999376 | 0.48 |
| rs5922893 | 83029159 | 0.42 |
| rs923865 | 83037453 | 0.40 |
| rs1114651 | 83048332 | 1.00 |
| **rs5968255** | **83060837** | |
| rs12845961 | 83095700 | 0.42 |
| rs2340868 | 83103157 | 0.42 |
| rs12391721 | 83117776 | 1.00 |
| rs12559134 | 83144695 | 0.45 |
| rs5967469 | 83156408 | 0.42 |
| rs9887469 | 83169279 | 1.00 |
| rs5922915 | 83174434 | 0.42 |
| rs1389465 | 83182794 | 0.42 |
| rs11092122 | 83187090 | 0.40 |
| rs10218349 | 83195648 | 1.00 |
| rs5016633 | 83199377 | 1.00 |
| rs1539517 | 83209323 | 0.42 |
| rs12556543 | 83248330 | 0.43 |
| rs2092545 | 83518130 | See Text |

**Table 5 P-values for the genotype and allele differences in females of the HapMap populations (Fisher test)**

| CEU vs. others | | |
|---|---|---|
| | Genotype (*P*) | Allele (*P*) |
| CEU_vs_CHB | 8.791322e-05 | 8.821520e-06 |
| CEU_vs_JPT | 1.103148e-05 | 1.123431e-06 |
| CEU_vs_YRI | 1.218292e-02 | 1.658306e-02 |
| | | |

| CHB vs. others | | |
|---|---|---|
| | Genotype (*P*) | Allele (*P*) |
| CHB_vs_CEU | 8.791322e-05 | 8.821520e-06 |
| CHB_vs_JPT | 1.000000e+00 | 6.733425e-01 |
| CHB_vs_YRI | 8.555027e-09 | 2.894914e-11 |

**Table 6 P-values for the genotype and allele differences in males of the HapMap populations (Fisher test)**

| CEU vs. others | | |
|---|---|---|
| | Genotype (*P*) | Allele (*P*) |
| CEU_vs_CHB | 0.0372554209 | 0.0372554209 |
| CEU_vs_JPT | 0.0006709185 | 0.0006709185 |
| CEU_vs_YRI | 0.6119536128 | 0.6119536128 |
| | | |

| CHB vs. others | | |
|---|---|---|
| | Genotype (*P*) | Allele (*P*) |
| CHB_vs_CEU | 0.037255421 | 0.037255421 |
| CHB_vs_JPT | 0.364044296 | 0.364044296 |
| CHB_vs_YRI | 0.002742678 | 0.002742678 |

### References

- 1.: S. J. O'Brien, G. W. Nelson, Nat Genet 36, 565 (Jun, 2004).
- 2.: A. L. Brass et al., Science 319, 921 (Feb 15, 2008).
- 3.: J. Fellay et al., Science 317, 944 (Aug 17, 2007).
- 4.: E. Gonzalez et al., Proc Natl Acad Sci U S A 98, 5199 (Apr 24, 2001).
- 5.: E. Gonzalez et al., Science 307,1434 (Mar 4, 2005).
- 6.: E. N. Fish, Nat Rev Immunol 8, 737 (Sep, 2008).
- 7.: K. E. Squires, Gend Med 4, 294 (Dec, 2007).
- 8.: R. K. Ahmed, G. Biberfeld, R. Thorstensson, Mol Immunol 42, 251 (Feb, 2005).
- 9.: "Materials and methods are available as supporting material on Science online".
- 10.: J. Rogers et al., Genomics 87, 30 (Jan, 2006).
- 11.: D. H. O'Connor et al., J Virol 77, 9029 (Aug, 2003).
- 12.: K. Szafranski, N. Jahn, M. Platzer, Bioinformatics 22, 1917 (Aug 1, 2006).
- 13.: A. Carriere, H. Ray, J. Blenis, P. P. Roux, Front Biosci 13, 4258 (2008).
- 14.: J. Xing, D. D. Ginty, M. E. Greenberg, Science 273, 959 (Aug 16, 1996).
- 15.: Nature 426, 789 (Dec 18, 2003).
- 16.: Nature 437,1299 (Oct 27, 2005).
- 17.: A. Amadori et al., Nat Med 1, 1279 (Dec, 1995).
- 18.: U. Sauermann et al., J Infect Dis 182, 716 (Sep, 2000).
- 19.: C. L. Qiu, H. Zhao, G. B. Yang, Q. Liu, Y. Shao, Vet Immunol Immunopathol 124, 313 (Aug 15, 2008).
- 20.: U. Sauermann et al., Genes Immun 9, 69 (Jan, 2008).
- 21.: V. M. Hirsch et al., J Virol 78, 275 (Jan, 2004).
- 22.: J. E. Wigginton, D. J. Cutler, G. R. Abecasis, Am J Hum Genet 76, 887 (May, 2005).
- 23.: "R Development Core Team. (R Foundation for Statistical Computing, Vienna, Austria, 2008)".
- 24.: A. L. Price et al., Nat Genet 38, 904 (Aug, 2006).
- 25.: O. Lao et al., Curr Biol 18, 1241 (Aug 26, 2008).
- 26.: R Development Core Team. (R Foundation for Statistical Computing, Vienna, Austria, 2008).
- 27.: J. C. Barrett, B. Fry, J. Maller, M. J. Daly, Bioinformatics 21, 263 (Jan 15, 2005).
- 28.: C. S. Carlson et al., Am JHum Genet 74, 106 (Jan, 2004).
- 29.: D. Ge et al., Genome Res 18, 640 (Apr, 2008).
- 30.: T. Muhl, M. Krawczak, P. Ten Haaft, G. Hunsmann, U. Sauermann, J Immunol 169,3438 (Sep 15, 2002).
- 31.: R. Pal et al., J Virol 76, 292 (Jan, 2002).
- 32.: Dummler, B.A., et al. The J. Bio.l Chem. 280,13304-13314. (2005)
- 33.: Thakur, A., et al. Clin. Cancer Res. 14, 4427-4436 (2008).
- 34.: Jiang, G., Espeseth, A., Hazuda, D.J., and Margolis, D.M. J. Virol. 81, 10914-10923 (2007).
- 35.: Connor, R.I., Sheridan, K.E., Ceradini, D., Choe, S., and Landau, N.R. (1997). The J. Exp. Med. 185, 621-628.
- 36.: Evans, M.J., von Hahn, T., Tscherne, D.M., Syder, A.J., Panis, M., Wolk, B., Hatziioannou, T., McKeating, J.A., Bieniasz, P.D., and Rice, C.M. Nature 446, 801-805 (2007).
- 37.: Lu, T.S., Avraham, H.K., Seng, S., Tachado, S.D., Koziel, H., Makriyannis, A., and Avraham, S. J. Immunol. 181, 6406-6416 (2008).
- 38.: Verma, S., Lo, Y., et al. Virology 385, 425-433 (2009)
- 39.: Zheng, J., et al. Retrovirology 2, 79 (2005).

## Claims

1. An in vitro method for diagnosing the likelihood and/or speed of AIDS progression in a mammal, comprising the analysis of at least one of the X-chromosomal SNPs from the *DXS986* region as defined by the SNPs rs825541 and rs2092545 in a sample obtained from said mammal.

2. The method according to claim 1, wherein said mammal is a mouse or human.

3. The method according to claim 1 or 2, wherein said SNPs are selected from the SNPs rs825541, rs1531868, rs5968255, rs10218349, and/or rs2092545, preferably rs5968255.

4. The method according to any of claims 1 to 3, wherein the presence of the rs5968255 C allele indicates a significant slower decline of the CD4⁺ T cell counts and lower viral loads in heterozygous HIV-1 infected females.

5. The method according to any of claims 1 to 4, wherein the r² of the SNP in relation to the SNP rs5968255 is higher than 0.4, preferably higher than 0.8, and most preferred higher than 0.95.

6. The method according to any of claims 1 to 5, wherein said analysis is accomplished by sequencing, mini-sequencing, hybridisation, restriction fragment analysis, oligonucleotide ligation assay or allele specific PCR.

7. The method according to any of claims 1 to 6, further comprising an analysis of the expression of at least one of the proteins CYLC1, HDX, POU3F4 and RPS6KA6, preferably RPS6KA6.

8. An in vitro method for screening for an anti-AIDS progression pharmaceutical compound, comprising the steps of:
a) measuring the expression of at least one of the proteins CYLC1, HDX, POU3F4 and RPS6KA6, preferably RPS6KA6, in cells from a mammal having an immunodeficiency virus-infection, preferably an HIV-infection, or in experimentally immunodeficiency virus-infected cells from a mammal,
b) contacting said protein *CYLC1, HDX, POU3F4* and/or *RPS6KA6,* with at least one potentially interacting compound, and
c) measuring the change of the expression of the protein CYLC1, HDX, POU3F4 and/or RPS6KA6 in the presence of said compound from step b).

9. The method according to claim 8, further comprising the steps of:
d) measuring the biological function of the protein CYLC1, HDX, POU3F4 and/or RPS6KA6, and/or the decline of the CD4⁺ T cell counts and/or viral load in the presence or absence of said binding compound.

10. The method according to claim 8 or 9, further comprising the step of performing a method according to any of claims 1 to 6.

11. A method for producing a pharmaceutical composition for the treatment of AIDS and/or AIDS progression, comprising the method according to any of claims 8 to 10, and further comprising the step of admixing said compound changing the expression of the proteins HDX, POU3F4, CYLC1 and/or RPS6KA6 with suitable auxiliary substances and/or additives.

12. Pharmaceutical composition for the treatment of AIDS and/or AIDS progression, produced according to claim 11.

13. A kit, in particular a diagnostic kit comprising materials for performing the method according to any of claims 1 to 10, in particular materials for analysing SNPs in a mammal that are selected from the SNPs rs825541, rs1531868, rs5968255, rs10218349, and/or rs2092545, and preferably rs5968255.

14. The kit according to claim 13, further comprising materials for analysing the proteins CYLC1, HDX, POU3F4 and/or RPS6KA6, in particular for an antibody-mediated detection or the detection of a modification of said proteins.
